# EUROPEAN PATENT APPLICATION

(11) **EP 4 700 013 A1**
(43) Date of publication of application: **25.02.2026**
(21) Application number: 24195474.2
(22) Date of filing: 20.08.2024
(51) Int. Cl.: C07C 273/04, C07C 273/16, B01J 19/00

(54) **UREA PRODUCTION PROCESS AND PLANT WITH MEDIUM-PRESSURE RECOVERY**

(71) Applicant: CASALE SA, 6900 Lugano (CH)
(72) Inventor: MARRONE, Leonardo, 21020 Mercallo (VA) (IT); BENEDETTI, Alberto, 22100 Como (IT)
(74) Representative: M. Zardi & Co S.A.

(57) **Abstract**

A urea production process including reacting ammonia and carbon dioxide in a high-pressure urea reactor (14), processing a reaction effluent (24) in a medium-pressure section (31, 131, 350) including carbamate decomposition and condensation of vapours of ammonia and carbon dioxide; condensation of said vapours is performed at a low temperature resulting in the formation of solid particles of ammonium carbamate; the so obtained condensation stream (109) is then subject to crystallization of ammonium carbamate producing a suspension (113) containing solid crystals of carbamate; at least part (115) of said suspension is recycled to the synthesis section as a liquid stream after melting the solid carbamate contained therein.

## Description

### Field of application

The invention is in the field of urea production. The invention relates to a process and plant for the urea production and to a method for modifying a urea production plant.

### Prior art

Urea is produced industrially by reacting ammonia and carbon dioxide at high temperature and high pressure. The reaction involves basically the formation of ammonium carbamate and its dehydration to form urea. The most employed process to produce urea is the stripping process. Another process of interest for urea production is the total-recycle process which is technically overcome by the stripping process but still in use, particularly for production of urea solutions suitable for NOx abatement systems, conventionally termed DEF (Diesel Exhaust Fluid) or AUS 32 according to ISO 22241. A detailed description of the urea production processes is available in Meessen, "Urea", in the Ullmann's Encyclopaedia of industrial chemistry 2012.

Essentially, urea is formed in a urea reactor at a high-pressure, usually more than 100 bar. A conversion of about 60% is achieved in the reactor, resulting in a mixture of urea, carbamate, water, CO2, and NH3 at the reactor outlet. In a stripping process, the reactor effluent is processed in a high-pressure stripper where ammonium carbamate is decomposed, ammonia and CO2 vapours are removed and condensed in a high-pressure condenser for recycle to the urea reactor. In a total-recycle process, there is no stripping and the effluent of the urea reactor is depressurized and sent directly to a downstream recovery section.

The synthesis effluent, which may be the effluent of the urea reactor or of the high-pressure stripper, is typically processed in one or more recovery sections to recover reagents contained therein. Typically, recovery is performed in a stand-alone low-pressure (LP) section or in a medium-pressure (MP) section followed by a low-pressure section. Generally, the medium pressure is around 18-25 bar and the low pressure is around 2-5 bar. Generally, but not necessarily, a CO2-stripping plant has only a low-pressure recovery section; other stripping plants and total-recycle plants feature also the medium-pressure section.

The one or more recovery section(s) produce a urea solution, typically with a concentration around 70%. This solution may be exported as such or subject to a finishing process for obtaining solid urea. The finishing process may include concentration of the solution to a urea melt and prilling or granulation of the melt. In an interesting application, the urea solution is diluted with water to obtain a diesel exhaust fluid (urea solution suitable for SCR of nitrogen oxides). If necessary, the solution may be processed to remove ammonia, in order to meet the quality requirements of the DEF.

A medium-pressure recovery section includes, typically:
a medium-pressure decomposer, where ammonium carbamate contained in the synthesis effluent is thermally decomposed to ammonia and carbon dioxide;
a first medium-pressure condenser and a distillation column, wherein a gaseous stream containing ammonia and carbon dioxide from the medium-pressure decomposer is subject to a partial condensation obtaining a carbamate liquid stream and ammonia vapours;
a second medium-pressure condenser where said ammonia vapours are further condensed obtaining an ammonia liquid stream;
a carbamate recycle line and an ammonia recycle line, which are separate lines for recycling said carbamate liquid stream and said ammonia liquid stream to the synthesis section.

The vapours emerging from the medium-pressure decomposer are partially condensed in a single-stage or two-stage condensation process. In a two-stage condensation process said vapours are first cooled in a heat exchanger, e.g. a shell-side vacuum pre-concentrator, to exploit the condensation heat to concentrate a urea solution flowing tube-side, then partially condensed to a temperature above 110 °C to avoid formation of solid crystals of carbamate. The partially condensed stream is sent to the distillation column to separate the carbamate stream from ammonia vapours; said ammonia vapours are then condensed to obtain an ammonia liquid stream. Said ammonia liquid stream is sent to the high-pressure synthesis section via an ammonia receiver where fresh ammonia is also introduced.

A drawback of the above technique is the capital cost of the medium-pressure section which requires many items, including separate pumps for recycling the carbamate and ammonia. This drawback is particularly relevant in urea plants of comparatively small capacity, such as 600 MTD (metric tons per day of urea) or less. As mentioned above, urea plants of such small capacity are still in use, particularly with the total-recycle process, and an interesting application of such plants is the production of DEF. There is therefore an incentive to reduce cost and complication of the medium-pressure section.

### Summary of the invention

The invention aims to simplify the medium-pressure recovery of urea production processes.

The aim is reached with a process according to the claims. Further aspects of the invention are a urea production plant and a method for modifying a urea production plant, as set out in the claims.

The invention is based on a novel approach to the step of condensation of the ammonia- and carbon dioxide-containing vapours obtained after the medium-pressure dissociation of the synthesis effluent. The condensation of said vapours is performed with a temperature of the condensation stream lower than usual, preferably not greater than 70 °C and more preferably not greater than 55°C, which is substantially lower than the conventional temperature of at least 110 °C. This temperature is selected so that the stream obtained after the condensation process contains solid particles (crystals) of ammonium carbamate. The condensation process allows to obtain a carbamate suspension containing solid crystals of carbamate, which is recycled to the synthesis section as a liquid stream after melting the solid carbamate contained therein.

The invention, therefore, goes against the conventional approach of avoiding crystallization of ammonium carbamate in the medium-pressure section. The invention deals with crystallization of carbamate throughout the condensation of the MP vapours. In a highly preferred embodiment of the invention, said condensation of vapours is a substantially total condensation (also termed quasi-total or nearly-total condensation) instead of a partial condensation, which is a further difference compared to the conventional condensation of vapours at medium pressure.

The invention eliminates the need of distillation and condensation of ammonia vapours, as well as the need of a separate line for recycling liquid ammonia, including a high-pressure ammonia pump. The related savings more than compensate for the cost of the specific equipment of the invention, such as crystallization section. Consequently, the complexity and cost of the medium-pressure section are reduced.

### Description of the invention

A process according to the invention includes reacting ammonia and carbon dioxide in a urea reactor of a high-pressure section operating at a high pressure; a synthesis effluent, containing urea, water and ammonium carbamate, is subject to processing at a medium pressure (MP); said medium-pressure processing includes: decomposition of ammonium carbamate contained in the liquid effluent in a medium-pressure decomposer, obtaining a purified urea solution and vapours of ammonia and carbon dioxide, and condensation of said vapours in a medium-pressure condenser.

Said condensation of the ammonia- and carbon dioxide-containing gaseous stream is performed to obtain a condensation stream at a temperature such that the condensation stream contains condensed ammonia and solid particles of ammonium carbamate.

Said condensation stream is collected in a vessel; a suspension, formed by the condensation stream is withdrawn from said vessel; at least part of said suspension is recycled to the synthesis section as a liquid stream after melting the solid ammonium carbamate contained therein.

Said vessel is preferably a crystallizer where the condensation stream is subject to a crystallization of ammonium carbamate and residual vapours are removed.

The temperature of said condensation stream, after the condensation process, is preferably not greater than 70 °C, preferably not greater than 55°C, more preferably between 40 and 50°C, to facilitate the formation of solid particles (crystals) of ammonium carbamate. Said temperature of the condensation stream after the condensation process may be the outlet temperature of said medium-pressure condenser.

Preferably, the condensation of the ammonia and CO2 vapours is a total condensation. Total condensation, also termed nearly-total or quasi-total condensation, denotes that vapours are completely condensed apart from a small fraction, which may be the inevitable residue of the condensation process. This is in contrast with partial condensation wherein a substantial fraction of vapours is deliberately not condensed.

In a preferred embodiment a nearly-total condensation is such that the mass fraction of the vapour (ratio between mass of residual vapours and total mass of the stream), after the condensation process, is less than 10% and more preferably less than 5%.

The condensation process of the MP vapours is preferably performed in two steps including a first step of partial condensation where no crystals are formed, followed by a second step of further cooling of the condensing stream with crystallization of ammonium carbamate in a medium-pressure crystallization section wherein residual vapours are condensed and a carbamate suspension containing solid crystals of carbamate is produced. Said second step leads preferably to a nearly-total condensation, as explained above. Preferably, at least part of said carbamate suspension is treated to melt the solid carbamate and to obtain a liquid stream, which is recycled to the synthesis section.

In an embodiment, MP vapours generated in a medium pressure decomposer are subjected to condensation in a MP condenser and then to crystallization in a MP crystallizer. In such case, the effluents of the crystallizer include a residual vapours stream and a carbamate suspension. The effluent of the MP condenser contains solid particles of carbamate formed during condensation; the crystallizer provides the required residence time to increase the size of said particles to form larger crystals.

In an interesting embodiment, a fresh ammonia stream is mixed with the condensation stream before said stream enters said vessel, for example MP crystallizer. In a preferred embodiment, said fresh ammonia stream has a temperature not higher than 40 °C, preferably not higher than 10 °C, more preferably not higher than -10 °C. In preferred embodiments the temperature of said ammonia stream is in the range -30 °C to +40 °C, preferably -30 °C to +10 °C and more preferably -30 °C to -10 °C

The carbamate suspension has preferably a solid content not higher than 50% by mass, or more preferably not higher than 40% by mass, or even more preferably not higher than 35% by mass. The solid content may be at least 1% or at least 5% or at least 10% by mass. Preferred ranges of solid content in the suspension are defined by the combinations of the above preferred low limits and upper limits.

A residence time of the suspension in said vessel or crystallizer is preferably at least 5 min, preferably 5 min to 10 min. A crystallizer may be designed accordingly, to provide the desired residence time.

A preferred embodiment provides a MP crystallization section including a shell and tube cooler operated at process-side outlet temperature below the crystallization point, and a buffer vessel. Preferably said buffer vessel is a MP crystallizer, properly designed to provide the required residence time to form and grow ammonium carbamate crystals.

Preferably, the carbamate suspension withdrawn from the vessel or from the crystallizer is the only stream effluent of the medium-pressure section that is recycled to the high-pressure section. As pointed out above, the invention does not require a separate condensation of ammonia vapours and recycle of liquid ammonia via an ammonia receiver and ammonia pump, which are found in the conventional technique.

Recycling of said carbamate suspension to the high-pressure section includes preferably a two-step compression comprising:
a first compression step wherein the carbamate suspension is brought to an intermediate pressure between the medium-pressure recovery section and the high-pressure synthesis section;
a first heating step, after said first compression, wherein the solid carbamate is melted, resulting in a liquid stream;
a second compression step wherein the liquid stream after the heating step is brought to a pressure suitable for reintroduction into the high-pressure section;
optionally, a second heating step after the second compression step.

In the first compression step, the pressure of the suspension is raised preferably by 5 to 30 bar, more preferably by 5 to 10 bar. The pressure after the first compression step is preferably at least 25 bar or at least 30 bar, such as 25 to 40 bar. A single-stage centrifugal pump may be suitable for this purpose. This first compression stage can be performed with a pump of easy procurement, e.g. a pump model 3HPX15 by Flowserve.

In the first heating step, the compressed suspension is heated to a temperature suitable for complete liquefaction of the crystals without reaching the boiling point of the liquid. Preferably said temperature is at least 60 °C and below the boiling point, more preferably in the range 60 to 100 °C or 60 to 80 °C or more preferably 70 to 80 °C.

Said two-step compression is preferred for processing a stream containing solid particles, in view of the significant difference between the high pressure, which is usually well above 100 bar, and the medium pressure, which is typically around 15-30 bar. Pumping a liquid/solid mixture is more challenging than pumping a liquid and a suitable pump may be more expensive; the invention addresses this issue by splitting between two compression steps.

Preferably, the pressure difference across the second compression step is greater than the pressure difference across the first compression step. The second compression step accounts preferably for a large majority, such as at least 70%, of the difference between the high pressure and the medium pressure of the process. The suspension is initially brought to intermediate pressure and then subject to heating in order to melt the solid phase, so that the following substantial compression is performed on a liquid. The advantage is that the first pump, processing a liquid/solid stream, has a relatively small pressure difference, whereas the large pressure difference is given by a pump working on a fully liquid stream.

The liquid stream obtained after the second compression and optionally after a second heating step, can be reintroduced in any item of the synthesis section. In an embodiment, said stream is introduced directly in the urea synthesis reactor. This may be the case of a total-recycle urea plant wherein the urea reactor is the main item of the synthesis section. In a stripping plant, said stream may be recycled e.g. to the high-pressure condenser.

In an embodiment, a stream of a clarified suspension is withdrawn from said vessel or crystallizer and is recycled to the MP condenser as aid to the condensation process. Said clarified suspension is an ammonia-rich liquid having a solid content reduced with respect to the suspension recycled to the urea synthesis. Said clarified suspension is preferably mixed with the stream entering the MP condenser so that the condensation completes and crystal nucleation process that here takes place is performed in the presence of said clarified suspension. Said recycle allows a reduction of solid concentration at the exchanger outlet preventing solid deposition on the tubes and therefore favouring condensation therein.

The MP condenser is preferably a shell-and-tube equipment, where the condensation of the process stream is performed in the tube side, and the shell side is traversed by a suitable cooling medium, such as cooling water. Preferably the process flow through this equipment is downflow to prevent solid build-up in the tubes. As stated above, said shell-and-tube MP condenser is preferably a quasi-total condenser of the MP vapours.

The urea solution effluent from the medium-pressure decomposer is sent, preferably, to a low-pressure recovery section. Said low-pressure recovery section produces an aqueous urea solution which may be exported as such or further processed, for example sent to an evaporation section to remove water for a subsequent finishing process (prilling or granulation), or processed to produce a DEF.

The vessel or the crystallizer may produce a stream of residual vapours which are preferably recycled to said low-pressure recovery section.

The medium pressure is preferably in the range 10 to 30 bar, more preferably 18 to 25 bar. The low pressure is preferably 2 to 6 bar. In this description and the attached claims, the pressure is given in bar gauge. The abbreviations MP and LP are used to denote the medium pressure and the low pressure.

The urea process can be of any type including particularly a total-recycle urea process or a stripping process. Preferably the invention is applied to a urea plant with a capacity lower than 600 MTD, more preferably lower than 400 MTD.

Further preferred embodiments of the invention are stated below.

Preferably, the process of the invention is conducted without an introduction of a passivating agent, typically air, and the high pressure is not greater than 160 bar. The absence of a passivating agent allows to avoid presence of inert in the MP section and thus minimize NH3 vapour slip from the MP crystallizer.

The MP crystallizer may comprise an upper compartment and a lower compartment arranged vertically, wherein the upper compartment is configured to allow separation of the residual vapours, and the lower compartment is configured to provide residence time needed by carbamate crystals to grow, the two compartments being in fluid communication with each other. If provided, the clarified suspension sent to the quasi-total MP condenser is taken from the upper portion of the lower compartment.

The lower compartment of the crystallizer has preferably a volume such the solid suspension of carbamate has a residence time in said lower compartment greater than 5 minutes, preferably from 10 to 40 minutes.

The MP crystallizer may comprise an external line to keep circulation of the carbamate suspension contained in said crystallizer. Accordingly, the crystallizer is kept stirred by circulating a portion of the carbamate suspension with said external line, so to guarantee solids are continuously kept in a fluidized state.

The crystallizer is preferably free of moving mechanical means, for example the crystallizer has no stirring means, to avoid sealing problems. Particularly preferably, the crystallizer is of the Oslo/Krystal type which promotes crystal settling. A detailed description of the Oslo/Krystal crystallizer is available in Perry's Chemical Engineers' Handbook, 2019, chapter 18-43.

In an embodiment, the vapour stream from the MP decomposer is partially condensed before being introduced in the MP condenser (which is preferably a quasi-total condenser). In an embodiment this preliminary step of partial condensation takes place in a process-to-process heat exchanger arranged to transfer heat from said vapour stream to another process fluid. More preferably, said process-to-process heat exchanger is arranged to transfer heat from said vapour stream to the compressed liquid recycled to the synthesis section.

In some embodiments, the plant does not include a wastewater treatment section and/or a purification section, and produces a urea solution suitable for DEF production by simple dilution of the urea solution with water.

### Brief description of the figures

Figure 1 represents a scheme of a conventional total recycle process for urea production.
Figure 2 represents a scheme of a urea production process according to an embodiment of the invention.
Figure 3 shows a detail of the MP condensation section according to an embodiment of the invention.

### Detailed description of figures

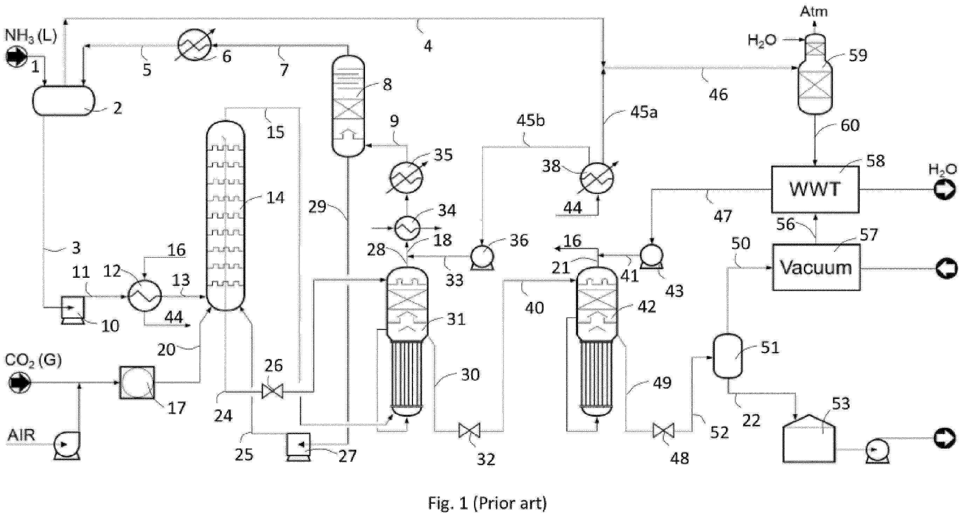

In Fig. 1, a fresh ammonia stream 1 is fed into an ammonia receiver tank 2. An ammonia stream 3, effluent of the ammonia receiver tank 2, is pumped through an ammonia pump 10 to a high-pressure (HP) such as 180 to 210 bar. The resulting pumped ammonia feed 11 is heated in a heat exchanger 12 by cooling a low-pressure (LP) stream 16 (which is described below) resulting in an ammonia feed 13. Said ammonia feed 13 is fed into the urea reactor 14, together with a carbamate recycle stream 25 and a CO2/air stream 20 compressed in a compressor 17. Inside the urea reactor 14, ammonia and carbon dioxide are reacted at the high pressure, obtaining a gaseous effluent 15 and a liquid effluent 24 containing mainly urea, water and carbamate.

The liquid effluent 24 of the reactor 14, which is a solution of mainly urea and carbamate, is expanded to a medium pressure (MP) of 18-25 bar in a valve 26, and is fed into a MP decomposer 31 with the gaseous effluent 15 of the urea reactor 14.

Inside the MP decomposer 31, a thermal decomposition of the carbamate contained in the liquid effluent 24 occurs, producing a urea solution 30 and a gaseous stream 28 containing NH3 and CO2. Said gaseous stream 28 is mixed with a LP recycle stream 33 and the resulting stream 18 is cooled in a two-stage heat exchange 34 and 35, to condensate most of the carbamate contained in the stream 18.

The cooled stream 9, comprising ammonia and carbamate, is treated in a MP absorber 8 to separate a carbamate liquid stream 29 from an ammonia rich vapour 7. The carbamate liquid stream 29 is pumped through a carbamate pump 27 and the resulting stream 25 is recycled in the urea reactor 14, whereas the ammonia rich vapour 7 is condensed in a condenser 6 and the resulting condensed ammonia 5 is sent to the NH3 receiver tank 2.

The urea solution 30 effluent from the MP decomposer 31 is expanded in a valve 32 from the medium pressure (18-25 bar) to a low pressure (2-5 bar), resulting in a urea solution 40 that is sent to the LP decomposer 42, wherein carbamate contained in the urea solution 40 is further decomposed and a purified urea solution 49 is formed. A LP gaseous stream 21 containing CO2 and NH3 produced by the decomposition of carbamate is withdrawn from the LP decomposer 42.

Said LP gaseous stream 21 is joined with a recycle stream 41, coming from a waste water treatment (WWT) section 58 (via line 47 and pump 43), to obtain the LP stream 16 cooled in the heat exchanger 12 by heating the pumped ammonia feed 11.

The resulting LP cooled stream 44 is condensed in an evaporator 38, wherein the heat removed from the stream 44 is employed to produce low-pressure steam, resulting in a LP condensate effluent 45. The LP condensate effluent 45 is, in part 45b, pumped in a pump 36 to obtain the LP recycle stream 33, and, in another part 45a, joined with an ammonia-rich stream 4 withdrawn from the NH3 receiver tank 2 to form a stream 46 to be washed. The stream 46 is fed into a LP absorber 59 wherein washing with water occurs, removing mainly ammonia and carbamate and producing an offgas vented in atmosphere and a wastewater stream 60.

The purified urea solution 49 is further expanded to a pressure lower than the atmospheric pressure in a valve 48, resulting in an expanded purified urea solution 52 which is flashed in a flash vessel 51 to separate a water rich vapour 50 and a concentrated urea solution 22. The concentrated urea solution 22 is the main product of the process, it is stored in a buffer tank 53 and can be sent to further evaporation steps to further remove water from urea if needed. The water rich vapour 50 is condensed in a vacuum condensation unit 57, resulting in a wastewater condensate 56 to be treated in the wastewater treatment section 58, together with the wastewater stream 60 effluent of the LP absorber 59.

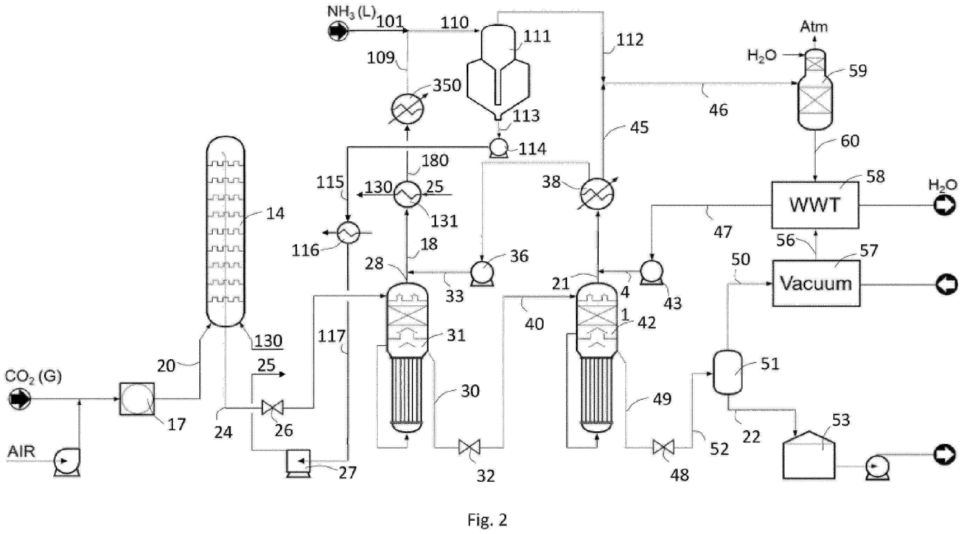

Fig. 2 represents the scheme of Fig. 1 modified in accordance with an embodiment of the invention. The gaseous stream 28, effluent of the MP decomposer 31, is joined with the LP recycle stream 33 and then cooled in a first process-to-process heat exchanger 131 resulting in a partially condensed stream 180. Heat removal in the heat exchanger 131 is provided by heating the carbamate solution 25 to form a heated carbamate solution 130 then recycled in the urea reactor 14.

The partially condensed stream 180 is fed into a quasi-total MP condenser 350 wherein the stream 180 is further cooled to a temperature below 70 °C, and ammonia and carbamate are respectively condensed and solidified, producing a first carbamate containing stream 109 that is further cooled thanks to an addition of a fresh ammonia stream 101 at a temperature below 40 °C. The resulting cooled carbamate melt 110 is sent to a crystallizer 111 wherein residual vapours 112 are removed and joined with the LP condensate effluent 45. Carbamate crystal growth occurs in the crystallizer 111, resulting in a carbamate suspension 113, which is recycled to the reactor 14.

The suspension 113 is pumped in a first compression stage 114; the resulting first compressed stream 115 is heated in a carbamate melter 116 wherein all the carbamate crystals are melted, producing a heated liquid stream 117; said liquid stream 117 is raised to synthesis pressure by a carbamate pump 27 and sent to the reactor, possibly with a second heating step in the exchanger 131.

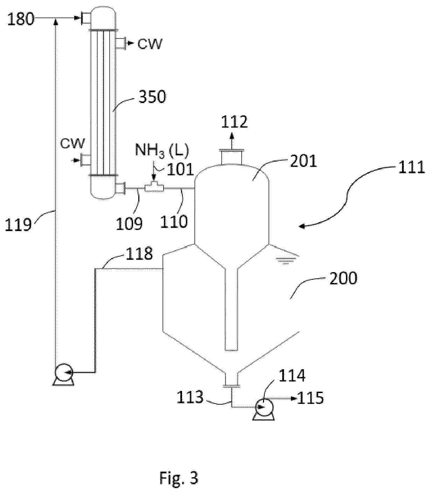

A detail of the MP condensation section according to a preferred embodiment of is shown in Fig. 3. The crystallizer 111 comprises an upper compartment 201 wherein residual vapours 112 are separated, and a lower compartment 200 designed to provide the suitable residence time for carbamate crystals to grow. The MP condenser 350 is a vertical shell-and-tube equipment.

The tube side of said condenser 350 is fed with the stream 180 added with an ammonia-rich liquid stream 118 withdrawn from the lower compartment 200 of the crystallizer 111. A cooling water flows shell-side in counter-current with respect to the process stream. The stream 180 is typically a gaseous stream or a biphasic mixture; the ammonia-rich liquid stream 118 contains solid matter resulting in a multiphase mixture introduced tube side in the condenser 350.

The fresh ammonia feed 101 is joined with the stream 109, resulting in a further cooled carbamate melt 110 that is introduced in the crystallizer 111. The ammonia rich-liquid stream 118 is withdrawn from the top of the lower compartment 200. Said stream 118 has a reduced solid content with respect to the carbamate suspension 113, and is mixed through a line 119 with the stream 180 in order to reduce the solid concentration inside the tubes of the MP condenser 350 and to favour condensation.

## Claims

1. A urea production process including:
reacting ammonia and carbon dioxide in a urea reactor (14), said urea reactor (14) comprised in a high-pressure section operating at a high pressure;
an effluent (24) of the high-pressure section, containing urea, water and ammonium carbamate, is subject to processing at a medium pressure;
said medium-pressure processing includes: decomposition of ammonium carbamate contained in the liquid effluent (24) in a medium-pressure decomposer (31), obtaining a purified urea solution (30) and a gaseous stream (28) of ammonia and carbon dioxide, and condensation of said gaseous stream in a medium-pressure condenser (350);
wherein: said condensation of the ammonia- and carbon dioxide-containing gaseous stream is performed to obtain a condensation stream at a temperature such that said condensation stream (109) contains condensed ammonia and solid particles of ammonium carbamate;
said condensation stream (109) is collected in a vessel (111) and a suspension (113), formed by the condensation stream, is withdrawn from said vessel;
at least part of said carbamate suspension (113) is recycled to the synthesis section as a liquid stream after melting the solid ammonium carbamate contained therein.

2. A process according to claim 1 wherein said vessel (111) is a crystallizer where the condensation stream (109) is subject to a crystallization of ammonium carbamate and residual vapours (112) are removed.

3. A process according to claim 1 or 2 wherein the temperature of said condensation stream (109), after the condensation process, is not greater than 70 °C, preferably not greater than 50 °C, more preferably in the range 30 °C to 70 °C or 30 °C to 50 °C.

4. A process according to any of the previous claims wherein a fresh liquid ammonia stream (101) is mixed with said condensation stream (109) before entering said vessel (111).

5. A process according to claim 4 wherein said fresh ammonia stream (101) is at a temperature not higher than 40 °C, preferably not higher than 10 °C, more preferably not higher than -10 °C, such as in the range -30 °C to -10 °C.

6. A process according to any of the previous claims wherein said carbamate suspension (113) has a solid content not higher than 50% by mass, preferably not higher than 40% by mass, and more preferably not higher than 35% by mass.

7. A process according to any of the previous claims wherein a residence time of the suspension in said vessel is at least 5 min, preferably 5 to 10 min.

8. A process according to any of the previous claims wherein said carbamate suspension (113) withdrawn from the vessel is the only stream effluent of the medium-pressure section that is recycled to the high-pressure section.

9. A process according any of the previous claims wherein recycling of said carbamate suspension (113) to the high-pressure section includes:
a first compression step (114) wherein the carbamate suspension (113) is brought to an intermediate pressure between the medium-pressure recovery section and the high-pressure synthesis section;
a heating step (116), after said first compression, wherein the solid carbamate is melted, resulting in a liquid stream (117);
a second compression step (27) wherein the liquid stream after the heating step (116) is brought to a pressure suitable for reintroduction into the high-pressure section.

10. A process according to claim 9 wherein: after the first compression step (114), the suspension has a pressure of at least 25 bar and preferably at least 30 bar, and during the heating step (116) the compressed stream is heated to a temperature of at least 60 °C and below the boiling point of the liquid, preferably to a temperature in the range 70 to 80 °C.

11. A process according to claim 9 or 10, wherein recycling of the carbamate suspension (113) to the high-pressure section includes a second heating step (131) after the second compression step (27).

12. A process according to any of claims 9 to 11, wherein said liquid stream (25), obtained after the second compression step (27), is reintroduced directly in a urea synthesis reactor (14).

13. A process according to any of the previous claims wherein a stream of a clarified suspension (118) is withdrawn from the vessel (111), said clarified suspension having a solid content reduced with respect to the suspension (113) recycled to the urea synthesis, said clarified suspension being recycled to said medium-pressure condenser (350) mixed with the gaseous stream (28) of ammonia and carbon dioxide obtained from decomposition in the medium-pressure decomposer (31).

14. A process according to any of the previous claims wherein a stream of residual vapours (112) is withdrawn from the crystallizer (111) and recycled to a low-pressure recovery section.

15. A process according to any of the previous claims wherein said medium pressure is in the range 10 to 30 bar.

16. A process according to any of the previous claims wherein the urea process is any of: a total-recycle urea process, a stripping process.

17. A process according to any of the previous claims wherein the mass fraction of the residual vapours, after said condensation of the ammonia- and carbon dioxide-containing gaseous stream, is less than 10%, preferably less than 5%.

18. A urea production plant comprising:
a high-pressure section including a urea reactor (14) wherein ammonia and carbon dioxide are reacted at a high pressure, the high-pressure section producing a synthesis effluent (24) containing urea, water and ammonium carbamate,
a medium-pressure section arranged to receive and process said synthesis effluent (24), the medium-pressure section comprising:
a medium-pressure decomposer (31) where said synthesis effluent is processed to obtain decomposition of ammonium carbamate and formation of a purified urea solution (30) and a gaseous stream (28) containing ammonia and CO2;
a medium-pressure condenser (350), preferably a nearly total condenser, configured to condense ammonia and crystallize carbamate contained in said gaseous stream obtaining a condensation stream (109) containing condensed ammonia and solid particles of ammonium carbamate, preferably at a temperature not higher than 70 °C;
a vessel arranged to receive said condensation stream;
a line arranged to send at least a part of a carbamate suspension withdrawn from said vessel to the synthesis section as a liquid stream after melting the solid carbamate contained therein.

19. A urea plant according to claim 18 wherein said vessel is a crystallizer (111) which is configured to process said condensation stream (109) to separate residual vapours (112) and to produce a carbamate suspension (113) containing solid crystals of carbamate,

20. A urea plant according to claim 19 wherein the crystallizer (111) comprises an upper compartment (201) and a lower compartment (200) arranged vertically, wherein the upper compartment (201) is configured to allow separation of residual vapours (112), and the lower compartment is configured to provide residence time needed by carbamate crystals to grow, the two compartments being in fluid communication with each other.

21. A method for modifying a urea production plant wherein:
the urea production plant includes, originally, a high-pressure synthesis section and a medium-pressure recovery section arranged to process a synthesis effluent (24) of the synthesis section;
said medium-pressure recovery section includes:
a medium-pressure decomposer (31), where ammonium carbamate contained in the synthesis effluent (24) is decomposed to ammonia and carbon dioxide;
a first medium-pressure condenser (35) and a distillation column (8), wherein a gaseous stream (28) containing ammonia and carbon dioxide from the medium-pressure decomposer is subject to a partial condensation and distillation obtaining a carbamate liquid stream (29) and ammonia vapours (7);
a second medium-pressure condenser (6) where said ammonia vapours are further condensed obtaining an ammonia liquid stream (5);
a carbamate recycle line and an ammonia recycle line, which are separate lines for recycling said carbamate liquid stream (29) and said ammonia liquid stream (5) to the synthesis section;
the method including the steps of:
replacing said first medium-pressure condenser with a new or modified medium-pressure condenser (350), which is preferably a nearly-total condenser, configured to condense ammonia and crystallize carbamate contained in said gaseous stream (28) obtaining a condensation stream (109) containing condensed ammonia and solid particles of ammonium carbamate, preferably at a temperature not higher than 70 °C;
installing a vessel (111) which is configured to receive said condensation stream and has an outlet for a carbamate suspension (113) containing solid crystals of carbamate,
discontinuing operation of said second medium-pressure and said ammonia recycle line,
modifying said carbamate recycle line to recycle at least part of said carbamate suspension (113) withdrawn from the vessel to the synthesis section as a liquid stream after melting the solid carbamate contained therein.

22. A method according to claim 21 wherein said vessel is a crystallizer configured to process said condensation stream to separate residual vapours (112) and to produce a carbamate suspension (113) containing solid crystals of carbamate.
